# EUROPEAN PATENT SPECIFICATION

(11) **EP 2 940 091 B1**
(45) Date of publication and mention of the grant of the patent: **26.06.2019**
(21) Application number: 15166156.8
(22) Date of filing: 03.05.2015
(51) Int. Cl.: C09J 7/25, A61F 13/58

(54) **ROLL OF ADHESIVE TAPE FOR PACKAGING**
KLEBEBANDROLLE FÜR VERPACKUNGSZWECKE
ROULEAU DE RUBAN ADHÉSIF POUR L'EMBALLAGE

(30) Priority: 02.05.2014 IT BO20140252
(43) Date of publication of application: 04.11.2015
(60) Divisional application: 16172288.9
(73) Proprietor: MAGIS S.P.A., 50050 Cerreto Guidi (FI) (IT)
(72) Inventor: Mannucci, Vanni, I 50139 Firenze (IT); Marzi, Marco, 50053 Empoli (FI) (IT)
(74) Representative: Mincone, Antimo

(56) References cited:
- EP-A1- 1 923 438
- EP-A1- 2 169 024
- CA-A1- 1 059 095
- US-A1- 2005 208 298
- US-A1- 2007 172 620
- US-A1- 2012 282 837

## Description

The present invention relates to a roll of self-adhesive tape for packaging, sealing boxes, and similar uses as defined in claims 1

In their usual conformation, the adhesive tapes for packaging are made by wrapping, around a cardboard core, a tape of plastic material, usually polypropylene, having a side coated by an adhesive that must have an adhesive strength such as to permit the unwrapping of the roll (which happens in many cases also thanks to the presence of a release agent on the side opposite to the adhesived side) and the subsequent fastening action on the materials to be bonded with the tape.

EP-0599789B2 was been granted for a roll of self-adhesive tape for packaging formed by a tape in plastic material wound on a tubular core made of cardboard, in which the diameter of the cardboard core is of the order of 38,2mm, in which the length of the tape is between approximately 105m and 220m and the outer diameter of the roll is about 120mm.

The adhesive tape of EP-0599789B2, as the other self-adhesive tapes currently on the market, has some drawbacks which are brilliantly solved by the present invention, as will be better clarified in the following of the present description, in which will also be more evident the many advantages that characterize the self-adhesive tape of the invention.

US2012/282837A1 describes an adhesive tape, especially for wrapping cables, consisting of a preferably textile carrier and of a pressure-sensitive adhesive which is applied on at least one side of the carrier and is in the form of a dried polymer dispersion.

US2007/172620A1 describes an adhesive tape having a backing material applied to at least one side of which is an adhesive, in particular a pressure-sensitive adhesive, the backing material being composed of at least one film having at least one score extending in the machine direction of the adhesive tape.

Among the advantages of the invention can be mentioned the following:
- with the present invention it is possible to provide a self-adhesive tape which, at the same volume, or at the same roll diameter, extends for linear values greater than the adhesive tapes of the traditional type, with obvious advantages both for the end user (greater autonomy of use of the single roll) and for logistics (increased amount of square meters with equal volume);
- the tape in question has a greater mechanical resistance (for example to tensile stresses) compared to a conventional adhesive tape of the same weight per square meter of the support;
- the possibility to use a substrate structure having lower weights per square meter which creates an improved environmental impact, since with the tape of the present invention the waste materials are reduced (in terms of weight), and an optimization of the production processes is obtained, since can be actually used jumbo rolls longer, thereby reducing the productive downtime;
- the adhesive tape of the invention adheres better to the pack or the box to be closed, because, thanks to the reduced thickness of the substrate, it can copy in a better way the profile and the surface shape of the object to be coated; in this way is required, therefore, a smaller amount of glue for the same adhesive efficacy;
- the adhesive tape of the invention, in the case of use of acrylic glues which do not involve the use of release agents, does not require the treatment of the back to make it polar (this is required in many cases to make noiseless/little noisy the unwinding of the tape), as instead it is required in the case of adhesive tapes that have polypropylene as base structure;
- it is possible to print the present adhesive tape, when made with acrylic glue, also on the outer face (side opposite to the adhesive side) without any corona treatment;
- in the case of hot-melt glues there is a better anchorage of the release agent (release), necessary with this type of adhesive masses in order to allow it to unwind.

This result is achieved, according to the present invention, by adopting the idea of making an adhesive tape for packaging having the characteristics indicated in claim 1. Other features of the present invention are described in the dependent claims.

The advantages of the present invention will be more evident thanks to the description which follows, made with reference to the attached figures, to be understood in the sense of non limiting examples, in which:
- Fig.1 is a schematic perspective view of a roll of adhesive tape made in accordance with the invention;
- Fig. 2 is a side view of the example of Fig.1;
- Fig. 3 is an enlarged side view of a possible embodiment of a tape in accordance with the invention;
- Fig. 4 schematically shows a possible example of the production stages of an adhesive tape according to the invention.

The present invention relates to a roll of adhesive tape (marked with 1 in the drawings) that can be used for packaging, or to join together boxes, containers, their parts, etc ..

The roll (1) is substantially composed of a central core (2) and a tape (3) wound around the same core (2) to form the body or the useful part (33) of the roll.

The central core (2) can be realized in cardboard and can have a diameter (D2) variable between 20 and 160 mm.

The tape (3) is formed by a film (30) on which is made to adhere, for example coated, a layer of adhesive (31) that provides the adhesive power to tape. The width (L3) is variable depending on the intended use of the adhesive tape (3).

Advantageously, in accordance with the invention, the film that constitutes the support structure (30) of the tape it is polyethylene terephthalate or PET.

In particular it is a film (non textile) PET with a thickness (H30) comprised between 8 and 23 µm.

In a particularly effective embodiment the thickness (H30) can be comprised between 8 and 15 µm.

The layer of glue has a grammage comprised between 8 and 32 grams per square meter, with an embodiment particularly effective between 8 and 22 grams per square meter.

The diameter (D1) of the roll (1) can be variable and will be determined by the total diameter of the tape, by the diameter (D2) of the specific central core, and by the thickness of the chosen PET base as well as from that of the applied adhesive mass.

In the production procedure of the present invention, it is possible to use coils of material (PET film) having a length very high; this results in increased productivity by reduction in downtime or slowdowns caused by the change of the coil; furthermore, the need for a smaller number of coils determines a lower contribution of waste.

On the film that defines the base (30) of the belt (3) can be used different types of adhesive (31) to determine the adhesive power as for example acrylic glue or hot-melt (also called HM in the present description).

In the case of acrylic glues, compared to the known techniques, it is possible to print on the outer face (32) without any treatment, instead as it is necessary for traditional tapes that need, for example, a corona treatment.

Always using as adhesive an acrylic glue, the tape in PET of the invention does not need any treatment on the back to increase the polarity and thus obtain tapes noiseless / little noisy. In fact, the noise is determined by the vibrations of the film during unwinding. In traditional tapes with polypropylene supports, by increasing the polarity of the back (side opposite to the adhesive-coated) by means of corona treatment, the interaction between the adhesive mass and the back is consequently increased: this determines an harder but more homogeneous unwinding, which determines in turn a decrease of vibrations during unwinding and consequently a lower noise level (the so-called low noise or low noise tapes according to the noise level depending by the adhesive force and by the level of treatment). In the case of PET, which already has a high intrinsic polarity (measured in terms of wettability and generally> 40 DIN / cm) due to its chemical composition, it is not necessary to perform corona treatment. This gives clear productive and economic advantages and increases the work safety (the corona treatment in air, the most common, involves in fact the production of ozone which must be adequately removed by suction to protect the health of the operator).

When the film (30) is coated with an adhesive (31) of hot-melt type, a greater anchoring power of the release agent is obtained thanks to the higher polarity of the PET support, which, compared to other traditional supports, allows a greater anchoring of the release agent due to the interaction forces of various kinds, such as hydrogen bonds, Van Der Waals forces, etc..

In addition, the tape (3) can advantageously be printed using the so-called sandwich technique, i.e. with protected printing; in practice, the print is performed on one side (34) of the film (30) PET and subsequently on the same side the adhesive is applied. In this way, the printing is located between two layers firmly united to each other: the film (30) on one side and the glue (31) on the other.

From what expressed, the advantages of the present invention are evident because a tape with a support made with PET film shows several advantages compared to one that has a BOPP support.

Experimental tests performed by the Applicant have shown, inter alia, the following advantages:
- the breaking loads for a tape with PET film are similar to those with a BOPP substrate having a thicknesses 50% greater: in practice, a 15 µm PET film has a break load value similar to a BOPP film of 23 µm; a 12 µm PET film similar to that of 19 µm in BOPP, and a 23 µm has a value similar to a 35 µm BOPP;
- PET adhesive tapes have greater flatness and dimensional stability;
- PET adhesive tapes have higher temperature resistance, especially at high temperatures.

All this allows to utilize supports thinner, with many advantages:
- greater conformability to the support where the product is applied, thus facilitating the adhesion even in cases of imperfect pressure applied;
- greater autonomy in terms of linear meters, with the same diameter of the roll: this feature allows to maximize the productivity, reducing the replacements on the machine, and optimization the transport (less volume for the same square meters and cores);
- optimal easy cutting, being intermediate between that of a PVC tape (which is greatly deformed during the cut) and that of a tape BOPP with easy tear (which, instead, tears too easily).

Compared to PVC substrates additional advantages are to be found also in the environmental sustainability of the product: in fact the PVC tapes have production processes that cannot be defined as eco-friendly (both as regards the support and with regard to the adhesive mass). The tapes PET instead, having both acrylic and hot melt adhesive masses, show a much more limited environmental impact.

Particularly advantageous are the relations between the roll diameter and the length of the adhesive tape. In particular, for PET tapes with hot-melt glue neutral (not printed), it is advantageously usable as support a film of 12 µm; in this case the sleeve or core (2) it may be only of 50.8 mm (2 inches), so as to obtain a very good compactness / autonomy ratio; also with a PET film of 15 µm excellent compactness / autonomy ratio can be obtained using a core of 50.8 mm or 76.2 mm (2 inches or 3 inches). The weights of glue can be similar to those of the products in BOPP; consequently, also the performance of the adhesive mass tested in laboratory (tests of peel and tack for example), will be similar; the benefits of the thin support will make however greater the adhesive efficiency in practice, as specified above.

For tapes with printed hot melt adhesive it was particularly advantageous to use a support to be 15 µm with core of 50.8 mm or 76.2 mm (2 or 3 inches). The weights of glue are similar to those of the products in BOPP; consequently, also the performance of the adhesive mass tested in the laboratory (tests of peel and tack for example), will be similar; the benefits of the thin support will make however in practical terms greater the adhesive efficiency, as specified above.

In the case of tape with acrylic glue (neutral, printed and printable) is advantageous to use a support of 23 µm, with the aid of a primer (which optimizes adhesion of the adhesive to the support) and with a HT weight of glue, thus placing the product (taking in account also the technical advantages listed above) at the high end of the range of packaging products.

Another great advantage derives, for the printable type, by the fact that the tape not requires the corona treatment before printing, thanks to the intrinsic polarity of the substrate. We have to consider that the corona treatment in the printing stage from above is always a delicate stage and not desirable: in fact a too high treatment could lead to defects in the unwinding (with possible loss of glue on the back or a break in the unwinding), while a too low treatment could result in a non perfect anchorage of the printing (with possible set-off). Moreover, the corona treatment requires energy costs and submit the operator at the exposure to ozone (subproduct of corona treatment with air plasma).

The possibility of facilitated cutting and the high adhesion of the glue to the support, make the tape with acrylic glue (both neutral and printed) excellent alternative to PVC tapes which are very often used by manufacturers of furniture thanks to their characteristics of " removability "(also in presence of high adhesion) and ease of application (thanks to the facilitated cutting).

The measurable benefits of this invention are related, mainly, to the mechanical strength: PET has a breaking load which is 50% greater than the equivalent thickness of BOPP (according to methodology AFERA 5004). In practice, a tape with PET film of 23 µm offers a strength similar to those of a tape with BOPP support of 35 µm. And a tape with a PET 23 µm support is similar to a 23 µm in BOPP.

Regarding to the adhesiveness (peel test 90° on steel, according to methodology AFERA 5001) a greater adhesion can also be observed (increase of about 20%); this appears mainly due to the reduced thickness which slightly changes the angle of attack (which nominally would be 90° but is influenced by the thickness: a higher thickness reduces the effective angle in the release area) and facilitates the adhesion in the reduced time required by the test. From this result it can be concluded that the application on cardboard (carton sealing - closing boxes) will be better thanks to the reduced thickness that facilitates the adhesion.

Thanks to the present invention it is possible to provide rolls of tape having lengths extremely high in relation to the total diameter (D1) of the roll (1).

From the data of Table 1 are clear the advantages in terms of size of the rolls of the examples used. The first line refers to a tape with PET support of from 15 µm with 17 gsm of HM glue and core of 76.2 mm (3 inches); the second line refers to an equivalent tape with BOPP support of 23 µm with 17 gsm of HM glue and core of 76.2 mm (3 inches); the third line refers to a tape with PET support of 23 µm with 21 gsm of acrylic glue and core of 76.2 mm (3 inches); the fourth line relates to an equivalent tape with BOPP support of 35 µm with 21 gsm of acrylic glue and core of 76.2 mm (3 inches). The difference in length is clear.

**Table 1**

| Lenght | Thickness | Core(D2) | Gramm | Diam(D1) |
|---|---|---|---|---|
| (meters) | (µm) | (mm) | (g/mq) | (mm) |
| 90,0 | 15,0 | 76,2 | 17,0 | 97,3 |
| 72,0 | 23,0 | 76,2 | 17,0 | 97,3 |
| 90,0 | 23,0 | 76,2 | 21,0 | 104,1 |
| 70,8 | 35,0 | 76,2 | 21,0 | 104,2 |

In Table 2 it is shown how it is advantageous the tape (1) of the invention also using a core (2) of 25.4 mm (1 inch).

**Table 2**

| Lenght | Thickness | Core(D2) | Gramm | Diam(Dl) |
|---|---|---|---|---|
| (meters) | (µm) | (mm) | (g/mq) | (mm) |
| 90,0 | 15,0 | 25,4 | 17,0 | 65,6 |
| 72,0 | 23,0 | 25,4 | 17,0 | 65,6 |
| 90,0 | 23,0 | 25,4 | 21,0 | 75,4 |
| 70,8 | 35,0 | 25,4 | 21,0 | 75,4 |

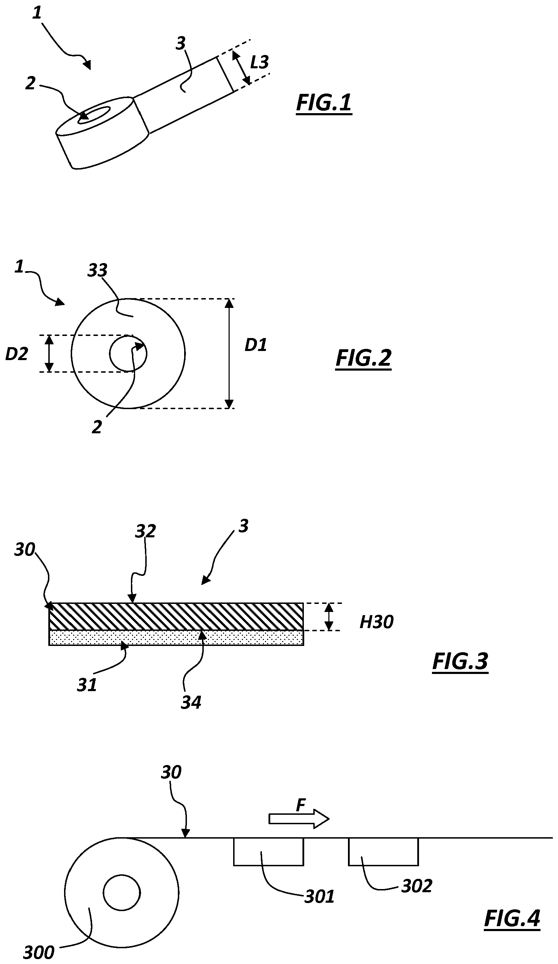

In Fig. 4 is schematically shown how to realize a tape in accordance with the invention. According to the invention, as specified in the independent claim of the patent description, the support of the tape is a PET film. The application of the tape is the packaging field (closing boxes and the like).

The process for the production of the roll of adhesive tape comprises the following steps: first unwinding from a reel (300) a Polyethylene terephthalate or PET film (non-textile). In Fig. 4 the arrow (F) indicates the direction of unwinding of the film. Thereafter, depending on the type of glue used, the process provides to apply a primer (301) in the case of acrylic glue or directly the glue in the case of hot-melt glue.

As described and illustrated has the value of an example of any modification or variant which falls within the protection defined by the claims.

## Claims

1. Roll of adhesive tape for packaging, of the type consisting of an inner core on which is wound a plastic tape which is made adhesive by means of the association of an adhesive, **characterized in that** said tape (3) is composed of a film (30) in Polyethylene terephthalate (PET) having a thickness comprised between 5 and 23 µm (microns) and an adhesive layer having a basis weight of between 8 and 32 grams per square meter.

2. Roll of tape according to claim 1 **characterized in that** the layer of glue is acrylic glue.

3. Roll of tape according to claim 1 **characterized in that** the glue layer is hot-melt glue.

4. Roll of tape according to claim 1 **characterized in that** the tape (3) is printed on the upper or external face (32) without treatment.

5. Roll of tape according to claim 1 **characterized in that** the tape (3) is printed by means of sandwich technique, i.e. with protected printing: the printing being carried out on one side (34) of the structure in PET (30) and subsequently on the same side is applied the adhesive, so that the printing is located between two layers firmly joined together: the base structure (30) on one side and the glue (31) on the other.

6. Roll of tape according to one of the preceding claims **characterized in that** the base structure (30) in PET has a thickness between 8 and 15 µm (microns).

7. Roll of tape according to one of the preceding claims **characterized in that** the glue layer has a basis weight of between 8 and 22 grams per square meter.

8. Roll of tape according to one of the preceding claims **characterized in that** the core (2) has a diameter (D2) of 76,2 mm and the roll (1) has a length of 90 m and an external diameter of 97,3mm and the glue layer has a basis weight of 17 grams per square meter.

9. Roll of tape according to one of the claims 1 to 7 **characterized in that** the core (2) has a diameter (D2) of 76,2 mm and the roll (1) has a length of 90 m and an external diameter of 104,1mm and the glue layer has a basis weight of 21 grams per square meter.

10. Roll of tape according to one of the claims 1 to 7 **characterized in that** the core (2) has a diameter (D2) of 25,4 mm and the roll (1) has a length of 90 m and an external diameter of 65,6mm and the glue layer has a basis weight of 17 grams per square meter.

11. Roll of tape according to one of the claims 1 to 7 **characterized in that** the core (2) has a diameter (D2) of 25,4 mm and the roll (1) has a length of 90 m and an external diameter of 75,4 mm and the glue layer has a basis weight of 21 grams per square meter.

12. Process for the production of a roll of adhesive tape according to one of the preceding claims, **characterized in that** it comprises the following stages:
a) unwinding a polyethylene terephthalate (PET) film from a reel (300);
b) in the case of acrylic glue, application of a primer (301), in the case of hot-melt glue, pass to the next step;
c) application of glue (302).

## Patentansprüche

1. Klebebandrolle zum Verpacken, von der Art bestehend aus einem inneren Kern, auf den ein Plastikband gewickelt ist, das mittels des Zusammenschlusses eines Klebstoffs klebend gemacht wird, **dadurch gekennzeichnet, dass** das Band (3) aus einer Folie (30) aus Polyethylenterephthalat (PET), die eine Dicke aufweist, die zwischen 5 und 23 µm (Mikrometer) liegt, und einer Klebstoffschicht, die ein Basisgewicht von zwischen 8 und 32 Gramm pro Quadratmeter aufweist, besteht.

2. Bandrolle nach Anspruch 1, **dadurch gekennzeichnet, dass** die Schicht von Klebstoff Acrylklebstoff ist.

3. Bandrolle nach Anspruch 1, **dadurch gekennzeichnet, dass** die Schicht von Klebstoff Schmelzklebstoff ist.

4. Bandrolle nach Anspruch 1, **dadurch gekennzeichnet, dass** das Band (3) ohne Behandlung auf der oberen oder äußeren Fläche (32) bedruckt ist.

5. Bandrolle nach Anspruch 1, **dadurch gekennzeichnet, dass** das Band (3) mittels einer Sandwich-Technik bedruckt ist, d.h. mit geschütztem Aufdruck: wobei das Drucken auf einer Seite (34) der Struktur aus PET (30) ausgeführt wird und anschließend auf derselben Seite der Klebstoff aufgebracht wird, so dass der Druck sich zwischen zwei fest zusammengefügten Schichten befindet: mit der Basisstruktur (30) auf einer Seite und dem Klebstoff (31) auf der anderen.

6. Bandrolle nach einem der vorstehenden Ansprüche, **dadurch gekennzeichnet, dass** die Basisstruktur (30) aus PET eine Dicke zwischen 8 und 15 µm (Mikrometer) aufweist.

7. Bandrolle nach einem der vorstehenden Ansprüche, **dadurch gekennzeichnet, dass** die Klebstoffschicht ein Basisgewischt von zwischen 8 und 22 Gramm pro Quadratmeter aufweist.

8. Bandrolle nach einem der vorstehenden Ansprüche, **dadurch gekennzeichnet, dass** der Kern (2) einen Durchmesser (D2) von 76,2 mm aufweist und die Rolle (1) eine Länge von 90 m und einen äußeren Durchmesser von 97,3 mm aufweist und die Klebstoffschicht ein Basisgewischt von 17 Gramm pro Quadratmeter aufweist.

9. Bandrolle nach einem der Ansprüche 1 bis 7, **dadurch gekennzeichnet, dass** der Kern (2) einen Durchmesser (D2) von 76,2 mm aufweist und die Rolle (1) eine Länge von 90 m und einen äußeren Durchmesser von 104,1 mm aufweist und die Klebstoffschicht ein Basisgewischt von 21 Gramm pro Quadratmeter aufweist.

10. Bandrolle nach einem der Ansprüche 1 bis 7, **dadurch gekennzeichnet, dass** der Kern (2) einen Durchmesser (D2) von 25,4 mm aufweist und die Rolle (1) eine Länge von 90 m und einen äußeren Durchmesser von 65,6 mm aufweist und die Klebstoffschicht ein Basisgewischt von 17 Gramm pro Quadratmeter aufweist.

11. Bandrolle nach einem der Ansprüche 1 bis 7, **dadurch gekennzeichnet, dass** der Kern (2) einen Durchmesser (D2) von 25,4 mm aufweist und die Rolle (1) eine Länge von 90 m und einen äußeren Durchmesser von 75,4 mm aufweist und die Klebstoffschicht ein Basisgewischt von 21 Gramm pro Quadratmeter aufweist.

12. Verfahren für die Herstellung einer Klebebandrolle nach einem der vorstehenden Ansprüche, **dadurch gekennzeichnet, dass** es die folgenden Schritte umfasst:
a) Abwickeln einer Folie aus Polyethylenterephthalat (PET) von einer Spule (300);
b) Im Falle von Acrylklebstoff, Aufbringen einer Grundierung (301), im Falle von Schmelzklebstoff, Weitergehen zum nächsten Schritt;
c) Aufbringen von Klebstoff (302).

## Revendications

1. Rouleau de ruban adhésif pour l'emballage, du type consistant en une âme interne sur laquelle est enroulé un ruban en plastique qui est rendu adhésif au moyen de l'association d'un adhésif, **caractérisé en ce que** ledit ruban (3) est composé d'un film (30) en poly(téréphtalate d'éthylène) (PET) ayant une épaisseur comprise entre 5 et 23 µm (microns) et d'une couche adhésive ayant un poids de base compris entre 8 et 32 grammes par mètre carré.

2. Rouleau de ruban selon la revendication 1, **caractérisé en ce que** la couche de colle est de la colle acrylique.

3. Rouleau de ruban selon la revendication 1, **caractérisé en ce que** la couche de colle est de la colle thermofusible.

4. Rouleau de ruban selon la revendication 1, **caractérisé en ce que** le ruban (3) est imprimé sur la face supérieure ou externe (32) sans traitement.

5. Rouleau de ruban selon la revendication 1, **caractérisé en ce que** le ruban (3) est imprimé au moyen d'une technique en sandwich, à savoir avec une impression protégée : l'impression étant effectuée sur un côté (34) de la structure en PET (30) et par la suite l'adhésif est appliqué sur le même côté, de sorte que l'impression est située entre deux couches solidement attachées ensemble : la structure de base (30) sur un côté et la colle (31) sur l'autre.

6. Rouleau de ruban selon l'une des revendications précédentes, **caractérisé en ce que** la structure de base (30) en PET a une épaisseur comprise entre 8 et 15 µm (microns).

7. Rouleau de ruban selon l'une des revendications précédentes, **caractérisé en ce que** la couche de colle a un poids de base compris entre 8 et 22 grammes par mètre carré.

8. Rouleau de ruban selon l'une des revendications précédentes, **caractérisé en ce que** l'âme (2) a un diamètre (D2) de 76,2 mm et le rouleau (1) a une longueur de 90 m et un diamètre externe de 97,3 mm et la couche de colle a un poids de base de 17 grammes par mètre carré.

9. Rouleau de ruban selon l'une des revendications 1 à 7, **caractérisé en ce que** l'âme (2) a un diamètre (D2) de 76,2 mm et le rouleau (1) a une longueur de 90 m et un diamètre externe de 104,1 mm et la couche de colle a un poids de base de 21 grammes par mètre carré.

10. Rouleau de ruban selon l'une des revendications 1 à 7, **caractérisé en ce que** l'âme (2) a un diamètre (D2) de 25,4 mm et le rouleau (1) a une longueur de 90 m et un diamètre externe de 65,6 mm et la couche de colle a un poids de base de 17 grammes par mètre carré.

11. Rouleau de ruban selon l'une des revendications 1 à 7, **caractérisé en ce que** l'âme (2) a un diamètre (D2) de 25,4 mm et le rouleau (1) a une longueur de 90 m et un diamètre externe de 75,4 mm et la couche de colle a un poids de base de 21 grammes par mètre carré.

12. Procédé pour la production d'un rouleau de ruban adhésif selon l'une des revendications précédentes, **caractérisé en ce qu'**il comprend les étapes suivantes :
a) le débobinage d'un film de poly(téréphtalate d'éthylène) (PET) à partir d'une bobine (300) ;
b) dans le cas d'une colle acrylique, l'application d'un primaire (301), dans le cas d'une colle chaude, le passage à l'étape suivante ;
c) l'application de colle (302).
